Europäisches Patentamt

European Patent Office

Office européen des brevets

(19)

(11) **EP 0 760 952 B1**

(12) **FASCICULE DE BREVET EUROPEEN**

(45) Date de publication et mention
de la délivrance du brevet:
**01.08.2001 Bulletin 2001/31**

(51) Int Cl.⁷: **G01N 33/543**, G01N 33/546,
G01N 33/553, G01N 35/00
// G01N33/569

(21) Numéro de dépôt: **96904987.3**

(22) Date de dépôt: **20.03.1996**

(86) Numéro de dépôt international:
**PCT/IB96/00245**

(87) Numéro de publication internationale:
**WO 96/29601 (26.09.1996 Gazette 1996/43)**

(54) **METHODE ET DISPOSITIF POUR LA DETERMINATION D'UN ANALYTE, NOTAMMENT D'UNE BACTERIE, PAR VOIE MAGNETIQUE**

VERFAHREN UND VORRICHTUNG ZUR BESTIMMUNG EINES ANALYTEN, INSBESONDERE EINES BAKTERIUMS, AUF MAGNETISCHEM WEGE

METHOD AND DEVICE FOR MAGNETICALLY DETERMINING AN ANALYTE, PARTICULARLY A BACTERIUM

(84) Etats contractants désignés:
**AT BE CH DE DK ES FR GB GR IE IT LI LU MC NL PT SE**

(30) Priorité: **21.03.1995 FR 9503528**

(43) Date de publication de la demande:
**12.03.1997 Bulletin 1997/11**

(73) Titulaire: **BIO MERIEUX**
**69280 Marcy l'Etoile (FR)**

(72) Inventeurs:
• **COLIN, Bruno**
**F-69280 Marcy-l'Etoile (FR)**

• **THERETZ, Alain**
**F-69130 Ecully (FR)**

(74) Mandataire: **Guerre, Dominique et al**
**Cabinet Germain et Maureau,**
**12, rue Boileau,**
**BP 6153**
**69466 Lyon Cedex 06 (FR)**

(56) Documents cités:
**EP-A- 0 339 623        EP-A- 0 357 786**
**US-A- 3 985 649        US-A- 4 913 883**

EP 0 760 952 B1

**Description**

La présente invention concerne la détermination qualitative et/ou quantitative d'un analyte susceptible d'être présent dans un échantillon.

Selon l'invention, par détermination d'un analyte, on entend la détection et/ou le dosage de toute substance biochimique ou biologique, et en particulier d'un matériel vivant, par exemple des cellules bactériennes ; dans ce dernier cas, la détermination quantitative peut consister en une numération des cellules bactériennes.

Mais selon la présente invention, l'analyte à déterminer peut être aussi, et sans limitation, un antigène, un haptène, un anticorps, un peptide, un fragment d'acide nucléique (ADN) ou (ARN), un enzyme, un substrat, à la condition que l'analyte considéré comprenne de manière générale un ligand ayant au moins un site de reconnaissance susceptible de se lier spécifiquement à un anti-ligand déterminé.

Les termes ligand et anti-ligand tels qu'utilisés dans la présente invention font référence à toutes molécules biologiques ou biochimiques susceptibles de former un complexe ligand/anti-ligand, tel que les complexes antigène/anticorps, anticorps/haptène, hormone/récepteur, protéine/anticorps, biotine/streptavidine, lectine/sucre, chélatant/molécule chélatée, hybride oligonucléotide/oligonucléotide, hybride olinucléotide/acide nucléique, enzyme/substrat ; étant entendu que lorsque le ligand est un fragment d'acide nucléique, il peut s'agir indifféremment d'un fragment d'ARN ou d'un ADN.

Par anticorps selon l'invention, on entend des anticorps monoclonaux, des anticorps polyclonaux, des fragments d'anticorps et des anticorps obtenus par recombinaison génétique.

Selon la demande de brevet EP-0 339 623, il est décrit et proposé un procédé pour la détermination d'un analyte dans un échantillon, selon lequel :

i) on dispose dudit échantillon en phase liquide, dont l'analyte comprend un ligand ayant au moins un site de reconnaissance spécifique d'un anti-ligand ;

ii) on dispose d'au moins un réactif comprenant des particules métalliques réactives, en suspension en phase liquide, et super-paramagnétiques, chaque particule réactive comprenant un noyau métallique sur lequel est fixé directement ou indirectement au moins ledit anti-ligand ;

iii) on met en contact, notamment par incubation, l'échantillon et le réactif, pour obtenir en phase liquide un mélange intermédiaire de complexes métalliques des particules réactives ayant réagi avec l'analyte, et des particules réactives résiduelles n'ayant pas réagi ;

iv) on applique un champ magnétique au mélange intermédiaire pour soumettre ce dernier à une magnéto-phorèse, ce qui provoque dans le mélange intermédiaire une migration différenciée des complexes métalliques, en phase liquide et sans séparation physique de ces derniers par rapport aux particules réactives résiduelles n'ayant pas réagi ;

v) on détecte en un point de référence du mélange intermédiaire, situé dans la direction du champ magnétique, et par diffraction de lumière, l'apparition des complexes métalliques, pour obtenir un signal de détection représentatif de la présence et/ou de la quantité desdits complexes, corrélé avec la présence et/ou la quantité d'analyte présente à l'origine dans l'échantillon.

Le procédé conforme au document EP-A-0 339 623 présente l'inconvénient de recourir à un moyen de détection des complexes métalliques, particulièrement compliqué, puisqu'en pratique il nécessite une source de lumière monochromatique cohérente (laser), un détecteur de la lumière diffractée, et un traitement du signal lumineux ainsi recueilli.

La présente invention a pour objet un procédé permettant une détection beaucoup plus simple des complexes métalliques.

Selon la présente invention, en combinaison :

a) selon l'étape (iv), avec le champ magnétique appliqué au mélange intermédiaire, d'une part on confine ou rassemble les complexes métalliques des particules réactives ayant réagi avec l'analyte, selon un agrégat de mesure, et d'autre part, on sépare, au sens d'une séparation physique,. l'agrégat de mesure des particules résiduelles n'ayant pas réagi ; le confinement et la séparation des complexes métalliques, par rapport aux particules résiduelles peuvent être effectués simultanément ;

b) selon l'étape (v), on détecte l'agrégat de mesure séparé, en mesurant sa masse ferro-magnétique, résultant de la réaction des particules métalliques réactives, super-paramagnétiques avec l'anti-ligand, et éventuellement de leur agglutination, cette masse ferro-magnétique étant représentative de la présence et/ou de la quantité totale des complexes métalliques.

Conformément à l'invention, on utilise de manière connue en soi, et comme dans le document EP-A-0 339 623, un réactif appelé notamment ferro-fluide, c'est-à-dire un réactif comprenant des particules métalliques réactives, en suspension en phase liquide, et super-paramagnétiques. Chaque particule réactive d'un tel réactif comprend un noyau métallique sur lequel est fixé, directement ou indirectement, au moins undit anti-ligand. La caractéristique essentielle d'un tel réactif est que dans l'état ou elles n'ont pas réagi avec le ligand, les particules métalliques réactives, magnétiques et/ou magnétisables, ne peuvent être séparées individuellement par voie magnétique de la phase liquide, et dans l'état ou elles ont réagi avec le ligand, elles sont ferro-magnétiques, et se comportent comme une masse ferro-magnétique, à l'état agrégé.

De tels réactifs, connus en soi, ont été décrits, complètement, y compris en ce qui concerne leur obtention, dans le document EP-A-0 339 823, et plus particulièrement dans les passages suivants de cette demande de brevet publiée :

- page 5, lignes 9 à 23 ;
- page 6, ligne 14, à page 7, ligne 13 ;

sans qu'il soit besoin d'incorporer d'autres développements ou explications dans la présente description.

Plusieurs modes de séparation de l'agrégat de mesure, par rapport au milieu intermédiaire dans lequel demeurent les particules résiduelles n'ayant pas réagi, peuvent être envisagés :

- on peut tout d'abord disposer, et notamment déplacer le champ magnétique lui-même, servant au confinement des complexes métalliques, pour déplacer ces derniers à partir et en dehors du milieu intermédiaire ;
- on peut ensuite fixer ou immobiliser le champ magnétique servant au confinement des complexes métalliques, et déplacer le milieu intermédiaire contenant les particules résiduelles à l'écart de l'agrégat de mesure ; pour ce faire, on peut, soit faire circuler le milieu intermédiaire par rapport au champ magnétique fixe, servant au confinement des complexes métalliques, soit prélever le milieu intermédiaire, notamment par pompage ou absorption dans un milieu absorbant, par rapport à l'agrégat de mesure retenu par le champ magnétique de confinement des complexes métalliques, ledit champ magnétique demeurant fixe comme dit précédemment.

Grâce à l'invention, la détection des complexes métalliques devient extrêmement simple, puisqu'il suffit de mesurer la masse ferro-magnétique de l'agrégat de mesure. A cette fin, différents moyens peuvent être utilisés, bien connus de l'homme du métier. Préférentiellement, on présente l'agrégat de mesure devant un capteur électro-magnétique, générant un signal électrique de mesure, représentatif de la masse ferro-magnétique, et par conséquent de la présence et/ou de la quantité des complexes métalliques formés entre le réactif et l'analyte.

La détermination quantitative de l'analyte peut être effectuée selon différents formats d'analyse, directe ou indirecte. Par exemple, dans une technique dite de compétition, on dispose d'un autre réactif dit de compétition, comprenant des particules métalliques réactives en suspension en phase liquide, super-paramagnétiques, chaque particule réactive comprenant un noyau métallique sur lequel est fixé directement ou indirectement un autre ligand spécifiquement reconnu par l'anti-ligand du réactif.

Un autre objet de l'invention est un dispositif pour la détermination qualitative et/ou quantitative d'un analyte dans un échantillon, comprenant :

i) un support, notamment un contenant pour recevoir ledit échantillon en phase liquide, dont l'analyte comprend un ligand ayant au moins un site de reconnaissance spécifique d'un anti-ligand ;
ii) une source d'au moins un réactif tel que précédemment défini, biologiquement réactif ;
iii) des moyens de mise en contact, notamment par incubation, de l'échantillon et du réactif, pour obtenir en phase liquide un mélange intermédiaire de complexes métalliques des particules réactives ayant réagi avec l'analyte, et des particules réactives résiduelles n'ayant pas réagi ;
iv) un moyen d'application d'un champ magnétique au mélange intermédiaire ;
v) un moyen de détection des complexes métalliques séparés, pour obtenir un signal de détection représentatif de la présence et/ou de la quantité desdits complexes, corrélé avec la présence et/ou la quantité d'analyte présente à l'origine dans l'échantillon.

Selon la présente invention, en combinaison :

a) le moyen d'application du champ magnétique selon (iv) comprend ou sert à la fois de moyen de confinement ou rassemblement des complexes métalliques entre les particules métalliques et l'analyte, selon l'agrégat de mesure, et de moyen de séparation (au sens d'une séparation physique) dudit agrégat, demeurant sur le support, par rapport aux particules résiduelles n'ayant pas réagi ;
b) le moyen de détection selon (v) comprend, d'une part un moyen de mesure de la masse ferro-magnétique, ou masse magnétique, de l'agrégat de mesure séparé, générant un signal de mesure représentatif de la présence et/ou de la quantité totale des complexes métalliques, et d'autre part un moyen de présentation du support avec l'agrégat de mesure, devant ledit moyen de mesure.

Les particules métalliques réactives en suspension en phase liquide sont constituées, comme dit précédemment, par un réactif, notamment ferro-fluide, sur lequel est fixé ou greffé, directement ou indirectement, ledit anti-ligand, par exemple un anticorps.

A titre d'exemple, le noyau métallique des particules du réactif est choisi parmi les matériaux paramagnétiques, qui sont intrinsèquement magnétiques et/ou magnétisables, tels que les sels complexes et les oxydes, les borures, les sulfures de fer, de cobalt, de nickel et les éléments de terres rares, ayant une susceptibilité magnétique élevée, tels que hématite et ferrites. Le noyau métallique des particules comprend des métaux purs ou des alliages comprenant un ou plusieurs de ces éléments. Le noyau métallique est dimensionné de préférence pour être dépourvu de magnétisme résiduel, et sa taille moyenne est comprise entre 5 et 30 nm, en particulier 10 et 20 nm. Le noyau métallique peut représenter de 5 à 100 % en poids, en particulier de 25 à 65 % en poids, de la particule réactive.

Les particules métalliques réactives peuvent comprendre une enveloppe en plus du noyau métallique. La composition de l'enveloppe n'est pas critique, dans la mesure où elle rend possible la fixation d'antiligands, et où elle est susceptible de présenter des interactions avec le noyau métallique. A titre d'exemple, l'enveloppe peut être un polymère naturel modifié ou non chimiquement, par exemple un polysaccharide tel que l'agarose, le dextrane et des dérivés de cellulose tels que la carboxyméthylcellulose; une protéine telle que la gélatine et un polymère d'albumine; un polymère de synthèse modifié ou non chimiquement tel que les acides acryliques ou méthacryliques.

La taille moyenne des particules réactives est comprise entre 20 et 100 nm, en particulier entre 50 et 70 nm.

Le procédé et le dispositif selon l'invention présentent un intérêt tout particulier pour la détermination d'un analyte, tel qu'une cellule bactérienne, en faible concentration dans un échantillon. En effet, à ce jour, il n'existe pas de méthode suffisamment sensible pour détecter directement quelques cellules bactériennes dans un échantillon, de sorte qu'il est nécessaire de recourir au préalable à une étape d'enrichissement.

Le ou les réactifs sont mélangés avec l'échantillon liquide qui est supposé contenir l'analyte et donc le ligand, puis le mélange est soumis par exemple à une incubation. La mise en contact de l'échantillon et du réactif est effectuée à un pH déterminé qui est fonction de la nature du ligand à détecter. Bien que la mise en contact puisse être exécutée dans une gamme de températures très large, de 2 à 95°C, la température avantageusement choisie est la température ambiante ou une température comprise entre 37°C et 40°C. Le temps de mise en contact est déterminé en fonction du type de détermination qualitative et/ou quantitative, selon des conditions opératoires conventionnelles connues en soi.

Habituellement, un tampon est utilisé pour maintenir le pH désiré. La solution tampon pouvant être utilisée peut être choisie parmi un tampon d'acide phosphorique ou un tampon Tris etc... Dans certains cas, des sels, des protéines ou des détergents sont ajoutés au mélange, du réactif et de l'échantillon, pour éviter des réactions non spécifiques.

Les principes à la base de la présente invention ont tout d'abord été explicités à partir du protocole expérimental décrit ci-après.

## 1) Synthèse de particules Fe$_3$O$_4$/Dextran T40 (50 %)

14 grammes de Dextran T40 (Mw = 40.000, Pharmacia) sont ajoutés à 14 ml d'eau, et on laisse se dissoudre le Dextran à température ambiante, pour former une première solution.

Une deuxième solution est préparée avec 3 grammes FeCl$_3$, 6H$_2$O (Mw = 270,30) et 1,3 grammes FeCl$_2$, 4H$_2$O (Mw = 198,81), dans 20 ml d'eau.

Les deux solutions sont introduites dans un réacteur double enveloppe de 250 ml, équipé d'un moteur d'agitation réglé à environ 200-250 tpm, d'un agitateur en verre et d'une ampoule de coulée contenant une solution de NH$_4$OH 7,5 % (v/v).

A température ambiante , on ajoute goutte à goutte la solution de NH$_4$OH 7,5 % (v/v) jusqu'à un pH final compris entre 10 et 11. Le réacteur est amené à 70°C pendant environ 60 minutes. A la fin de la réaction, la solution est récupérée et dialysée extensivement contre 5 l d'eau distillée, puis filtrée sur laine de quartz. La solution est ensuite centrifugée 3 fois à 600 tpm pendant 5 minutes.

Afin d'éliminer le Dextran qui n'a pas réagi, les particules sont déposées sur une colonne de 33 x 2,5 cm de gel de Sephacryl S300 HR (Pharmacia), préalablement équilibrée par un tampon acétate 0.1 M, NaCl 0.15 M, 0,05 % NaN$_3$, pH 6,5.

Les particules métalliques du ferro-fluide obtenu ont un diamètre extérieur compris entre 20 et 900 nm, et préférentiellement compris entre 50 et 70 nm, avec une enveloppe de Dextran. Elles sont conservées à +4°C.

## Préparation des particules réactives portant au moins un anticorps dirigé contre les *Listeria,* appelé ci-après Ac anti-L.

Les particules synthétisées selon l'Exemple 1 sont oxydées, par addition de périodate de sodium (0,1 M).

Après agitation pendant 45 minutes, à l'abri de la lumière, les particules sont soumises à une dialyse, contre du NaCl 0,15 M, pendant 4 heures.

A la solution contenant les particules métalliques, dont la concentration est de 1 mg de particules par mg de réactif, 10 μg/ml d'Ac anti-L sont ajoutés ; le pH est ajusté par addition de NaHCO$_3$ à environ 8.

Le mélange est incubé pendant 20 heures, sous agitation douce. Le mélange ainsi formé est réduit par addition d'une solution de borohydrure de sodium, selon une concentration de 12-10$^{-3}$ moles NaBH$_4$ par mg de particules, sous agitation douce pendant 45 minutes. Le mélange est dialysé toute la nuit contre un tampon phosphate de sodium (0,1 M)-NaCl (0,15 M) à pH 7,5.

La solution finale contient 0,415 mg de particules métalliques par ml de réactif, 15,4 μg d'Ac anti-L par mg de particules, soit environ 6,4 μg Ac anti-L par ml de réactif.

**Détermination qualitative et/ou quantitative, notamment détection de bactéries Listeria avec un capteur électromagnétique.**

Le montage expérimental utilisé comprend :

- un support 2, identique à celui d'une bande d'enregistrement audio ou vidéo traditionnelle, par exemple un film polyester, de 5/100$^{èmes}$ de mm d'épaisseur ; ce support consiste en une bande comprenant, d'une part, un talon de fixation 2b, et une languette 2a relativement libre, et en particulier susceptible d'être soumis à des oscillations, notamment de vibrer par tout moyen approprié, notamment sous l'effet d'un flux d'air tangentiel défini ci-après ; ce support imperméable est destiné à recevoir en phase liquide l'échantillon à traiter comprenant la bactérie à doser, et/ou le réactif préparé comme précédemment, et/ou le mélange intermédiaire comprenant les complexes métalliques consistant en des particules réactives ayant réagi avec les bactéries, considérées en tant qu'analyte ;
- un moyen fixe 21 permettant de fixer le support précédemment défini, à l'aide de deux crochets 23 par exemple ;
- un moyen 4 de détection des complexes métalliques, selon un agrégat de mesure confiné et séparé, tel que précédemment défini, supporté par le support 2 ; conformément à l'invention, ce moyen 4 de détection est un moyen de mesure de la masse ferro-magnétique de l'agrégat de mesure séparé, et comprend un capteur électromagnétique 6 générant un signal électrique de mesure, par exemple une force électro-motrice de détection, représentative de la masse ferro-magnétique de l'agrégat de mesure séparé, et par conséquent de la présence et/ou de la quantité totale de complexes métalliques ; ce capteur est constitué de manière traditionnelle, comme pour la tête de lecture d'une bande enregistrée audio ou vidéo, par un circuit magnétique 24 définissant un entrefer magnétique 12, à distance duquel et devant lequel est disposée la languette 2a du support 2 ; une bobine 20 est disposée entre les deux branches du circuit magnétique 24, ayant une forme en "U" en section transversale ;
- une chaîne de traitement du signal électrique de mesure à la sortie de la bobine 20, comprenant un amplificateur 18 dont le gain peut être réglé, et un afficheur 19 en sortie de l'amplificateur 18 ;
- un aimant permanent 11, ou générateur magnétique auxiliaire, situé au-dessus du capteur 6 électromagnétique, pour augmenter le signal de mesure, en sortie du capteur 6, et plus précisément de la bobine 20 ;
- un moyen 10 de mise en oscillation ou vibration, selon une direction verticale de la languette 2a, constitué par une buse 10, projetant un flux d'un gaz inerte focalisé sur la tranche ou le chant de la languette 2a ; l'intensité du flux gazeux ainsi projeté est réglée en contrôlant la pression du gaz dans la buse 10, au moyen d'un manomètre 22 ; le moyen 10 de mise en vibration peut être un moyen électromécanique.

En fonction de l'intensité d'un flux d'air projeté par la buse 10, mesurée selon une unité arbitraire, par exemple les graduations du manomètre 22, on mesure la tension de sortie 19, pour différentes configurations, à savoir :

- sans languette 2a devant et à distance de l'entrefer magnétique 12, soit $V_0$ ;
- en présence de la languette ou support 2a, mais sans réactif, toujours à distance et devant l'entrefer 12, soit $V_b$ ;
- et enfin, avec une quantité prédéterminée du réactif, devant et à distance de l'entrefer 12, soit V.

Pour ce premier essai, le gain de l'amplificateur 18 est fixé à la valeur de 4,2.

Quelle que soit l'intensité du flux d'air appliqué tangentiellement par rapport à l'entrefer 12, la tension affichée selon 19 demeure sensiblement la même, qu'un support 2a soit présent ou non devant l'entrefer 12.

A partir d'un flux d'air de 52 (valeur arbitraire correspondant à une pression d'environ 150 millibars), la tension affichée avec le support magnétique, soit "V", augmente fortement, puisqu'elle passe de 18 mV à 50 mV entre 11 et 51 pour le flux d'air, et de 50 à 190 mV à 52, pour atteindre un maximum à 62.

Conséquemment, la valeur du flux d'air retenu pour les essais suivants est voisine de 62.

Ensuite, pour un même flux d'air fixé à la valeur de 62, on détermine les tensions affichées $V_0$, $V_b$, maximum et minimum, en fonction du gain de l'amplificateur 18. On constate ainsi que pour détecter un signal significatif, le gain de l'amplificateur 18 doit être supérieur à 4.

Avec les deux valeurs ainsi déterminées, respectivement du flux d'air, soit 62, et du gain de l'amplificateur, soit 4, on procède ensuite à une optimisation du rapport signal/bruit, exprimée par la relation

$$\frac{V_m - V_o}{V_b - V_o}$$

dans laquelle $V_m$ est la tension affichée en 19 pour une mesure.

Tout d'abord, en utilisant un support 2a, on dépose sur cette dernière un μl de ferro-fluide, identique aux particules métalliques précédemment considérées, mais avant fixation de l'anti-ligand ou anticorps, et des bactéries E.Coli. En cours d'incubation, on constate que la tension mesurée $V_m$ demeure sensiblement constan-

te, et égale en moyenne à 11,95, à 1,1 % près, avec un écart type de 0,13. Cette tension n'est pas très différente des tensions $V_o$ et $V_b$ obtenues dans les mêmes conditions expérimentales, de telle sorte que le rapport signal/bruit n'est pas très important.

En faisant varier à nouveau le gain de l'amplificateur 18, on détermine que le rapport signal/bruit est optimum pour un gain de 6, en conservant la même intensité du flux d'air assurant la vibration du support 2a. Dans ces conditions, en utilisant un support 2a du type bande magnétique audio-vidéo, sur lequel est reçu un μl du réactif préparé précédemment, comprenant 0,5 mg par ml de ferro-fluide avec 0,5 μl par ml d'anticorps anti-Listeria, le rapport signal/bruit obtenu est de 140.

En conservant le même flux d'air et le même gain, avec un support 2a du type bande magnétique audio-vidéo, on mesure la différence entre la tension de mesure $V_m$ et la tension $V_b$, respectivement pour un μl d'eau, 1,46 ng de ferro-fluide non revêtu et non ligué à l'anticorps, 14,6 ng, 146 ng et 1,46 μg du même ferro-fluide ; et on obtient ainsi les valeurs rassemblées dans le tableau ci-dessous.

|          | $V_m$ | $V_m$-$V_b$ |
|----------|-------|-------------|
| $V_o$    | 10,2  |             |
| $V_b$    | 10,6  |             |
| eau      | 11,2  | 0,6         |
| 1,46 ng  | 10,7  | 0,1         |
| 14,6 ng  | 12,3  | 1,7         |
| 146 ng   | 11,9  | 1,3         |
| 1,46 μg  | 15,3  | 4,3         |

Il résulte de ce tableau que la plus petite quantité de réactif détectable est de 14,6 ng, étant observé que le signal affiché n'augmente pas proportionnellement à la quantité de réactif.

Toujours avec les mêmes conditions de flux d'air et de gain d'amplification, et un support 2 du type bande magnétique, on détermine successivement les tensions $V_o$, $V_b$ et $V_m$, ainsi que le rapport signal/bruit, lorsque le support comprend un mélange intermédiaire constitué par le complexe entre le réactif précédemment préparé et un échantillon en phase liquide contenant 10E9 par ml de bactéries Listeria. Dans ces conditions, la tension $V_m$ obtenue est de 530 mV et le rapport signal/bruit de 113. Ce rapport peut être augmenté en diminuant $V_b$, par exemple en diminuant l'intensité du flux d'air éjecté par la buse 10. Par exemple, et dans le cas des bactéries Listeria, le flux d'air peut être amené à une valeur de 44 (selon les unités arbitraires définies précédemment), pour obtenir le plus petit signal détectable avec les bactéries précitées. Dans ces nouvelles conditions, pour la même concentration en bactéries de l'échantillon en phase liquide, et avec un réactif présent sur le support, au contact de l'échantillon, dans la proportion de 5 μg/ml d'anticorps et de 1 mg/ml de ferro-fluide, on obtient un signal de sortie de 700 mV, avec un rapport signal/bruit de 1714.

Ce protocole expérimental démontre donc qu'il est possible d'obtenir selon l'invention un signal de sortie pouvant être corrélé à la présence et/ou la quantité ou nombre de bactéries présentes dans l'échantillon à doser ou détecter, donc un signal de mesure.

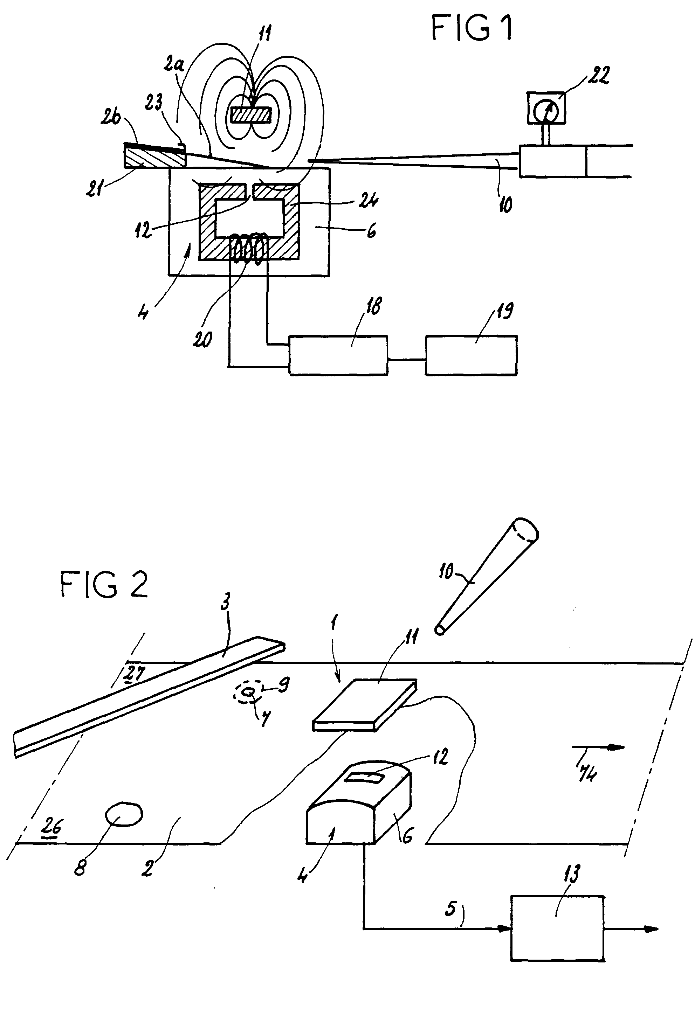

Conformément au dessin annexé, comprenant des figures 2 à 5 schématiques, sont illustrés différents dispositifs de détermination qualitative et/ou quantitative selon la présente invention.

Conformément à la figure 2, un dispositif 1 selon l'invention comprend :

i) un support 2, déplacé en translation selon la direction et le sens 74, imperméable, permettant de recevoir l'échantillon en phase liquide, par exemple un inoculum déposé sur le support 2 dans une zone de réception 8 ;

ii) une source du réactif défini précédemment étant disponible, pour mettre au contact ledit réactif avec ledit échantillon, par exemple dans la zone 8 ;

iii) cette mise en contact, notamment par incubation, de l'échantillon et du réactif, permettant d'obtenir en phase liquide, par exemple dans la zone 8, le mélange intermédiaire précédemment défini ;

iv) un moyen 3 d'application du champ magnétique servant à la fois de moyen de confinement des complexes métalliques précédemment définis selon un agrégat de mesure, et de moyen de séparation sur le support dudit agrégat, par rapport aux particules n'ayant pas réagi, ce moyen 3 consistant en un aimant permanent disposé transversalement par rapport au support 2 ayant la forme d'une bande ;

v) un moyen 4 de détection des complexes métalliques ainsi confinés et séparés, comprenant le capteur 6 électro-magnétique de la masse ferro-magnétique de l'agrégat de mesure, pour obtenir un signal 5 de mesure, pouvant être visualisé en 13, et représentatif de la masse magnétique de l'agrégat, et partant de la présence et/ou la quantité des complexes métalliques, laquelle peut être corrélée avec la quantité d'analyte présente à l'origine dans l'échantillon.

Selon la présente invention, et en combinaison :

a) le moyen 4 de détection selon (v) comprend, d'une part le moyen de mesure 6 de la masse ferro-magnétique générant le signal de mesure 5, de nature électrique, et représentatif de la présence et/ou de la quantité totale des complexes métalliques originellement présents dans le mélange intermédiaire précité ; et le support 2 en forme de bande constitue un moyen de présentation de ces complexes métalliques, confinés et séparés, par passage de ces derniers devant et à distance du capteur électromagnétique 6 , situé en dessous de la bande 2 ;

b) le moyen 3 d'application du champ magnétique selon (iv) ; en l'occurrence l'aimant permanent transversal, sert à la fois de moyen de confinement des complexes métalliques, en les rassemblant selon l'agrégat de mesure, et de moyen de séparation dudit agrégat en le transportant de la zone de réception 8 à une zone de séparation 9, pour obtenir dans cette dernière ledit agrégat à l'état séparé, lequel est alors susceptible d'être détecté par le capteur électromagnétique 6, par sa présentation ou passage à distance et devant ce capteur.

Comme dit précédemment, le support 2 est plat, et consiste par exemple en une bande imperméable vis-à-vis de l'échantillon, du réactif, et du mélange intermédiaire, en phase liquide. Sa surface supérieure est telle que la phase liquide des partenaires réactionnels puisse glisser ou couler d'une zone à une autre, par exemple de la première zone 8 recevant le mélange intermédiaire au moins, jusqu'à la deuxième zone 9 de séparation de l'agrégat de mesure, sous l'effet de l'aimant 3 générant un champ magnétique permettant notamment de transférer l'agrégat de mesure de la première zone à la deuxième zone.

Comme dans le montage expérimental décrit par référence à la figure 1, on génère des vibrations du support 2, selon une direction verticale par rapport au plan horizontal dudit support, lesquelles sont transmises à l'agrégat de mesure, au moment de son passage devant et à distance du capteur électromagnétique 6. Ce générateur peut consister comme décrit précédemment en un moyen de projection 10 d'un flux gazeux tangentiel à l'encontre de la tranche ou du chant du support 2.

Un générateur magnétique auxiliaire 11 est disposé au niveau du capteur électromagnétique 6, de manière à augmenter le signal de détection généré par ce dernier.

Le procédé de détermination qualitative et/ou quantitative d'un analyte, par exemple d'une bactérie, dans un échantillon, se déduit de la description précédente, et comprend les étapes suivantes :

i) on dispose l'échantillon en phase liquide dans la première zone 8 ;

ii) on apporte dans cette zone 8 le réactif défini précédemment ;

iii) par incubation, on laisse s'établir un contact entre l'échantillon et le réactif dans la première zone 8, pour obtenir en phase liquide le mélange intermédiaire comprenant, et les complexes métalliques, et les particules réactives résiduelles n'ayant pas réagi ;

iv) par le champ magnétique généré par l'aimant 3, et appliqué au mélange intermédiaire, on confine les complexes métalliques selon un agrégat de mesure, et on sépare ledit agrégat des particules n'ayant pas réagi, en transférant cet agrégat 7 de la première zone 8 à la deuxième zone 9, pour obtenir dans cette dernière un agrégat 7 de mesure, séparé, susceptible d'être détecté par le capteur électromagnétique 6, par passage devant et à distance de ce dernier ;

v) on détecte l'agrégat de mesure 7 ainsi séparé, en mesurant sa masse ferro-magnétique, par présentation dudit agrégat, devant le capteur électromagnétique 6, lequel génère un signal électrique de mesure 5 représentatif de la présence et/ou de la quantité ou nombre total des complexes métalliques précités.

Il existe donc sur le support 2, en mouvement en translation selon la flèche 74, en quelque sorte deux pistes, la première 26 réactionnelle, et la seconde de mesure 27, par rapport à laquelle le capteur électromagnétique 6 est disposé sous la bande ou support 2.

Comme décrit précédemment, grâce à l'aimant 3, les complexes métalliques sont confinés et séparés ou extraits sélectivement de la première zone 8, et transférés vers la deuxième zone de séparation 9, et par conséquent transférés en quelque sorte de la première piste 26 à la deuxième piste 27, et ceci sous l'effet du champ magnétique généré par l'aimant 3, focalisant les lignes magnétiques sur la deuxième zone 9. Ce transfert s'effectue à plat, sur le support ou bande 2.

Comme le support plat 2 est soumis à des oscillations périodiques, selon une direction verticale, sous l'effet du jet gazeux émis par la buse 10, rencontrant la tranche du support 2, l'agrégat de mesure 7 se trouve lui-même soumis à des oscillations ou vibrations, devant le capteur électromagnétique 6.

Et pour augmenter le signal de détection 5, on applique, grâce à l'aimant 11, un champ magnétique auxiliaire, au niveau du capteur 6. Un tel aimant est par exemple un aimant néodyme-fer-bore (diamètre 6 mm, hauteur 5 mm) dont la résonance est supérieure à 30 % et le produit énergétique est de 70 %.

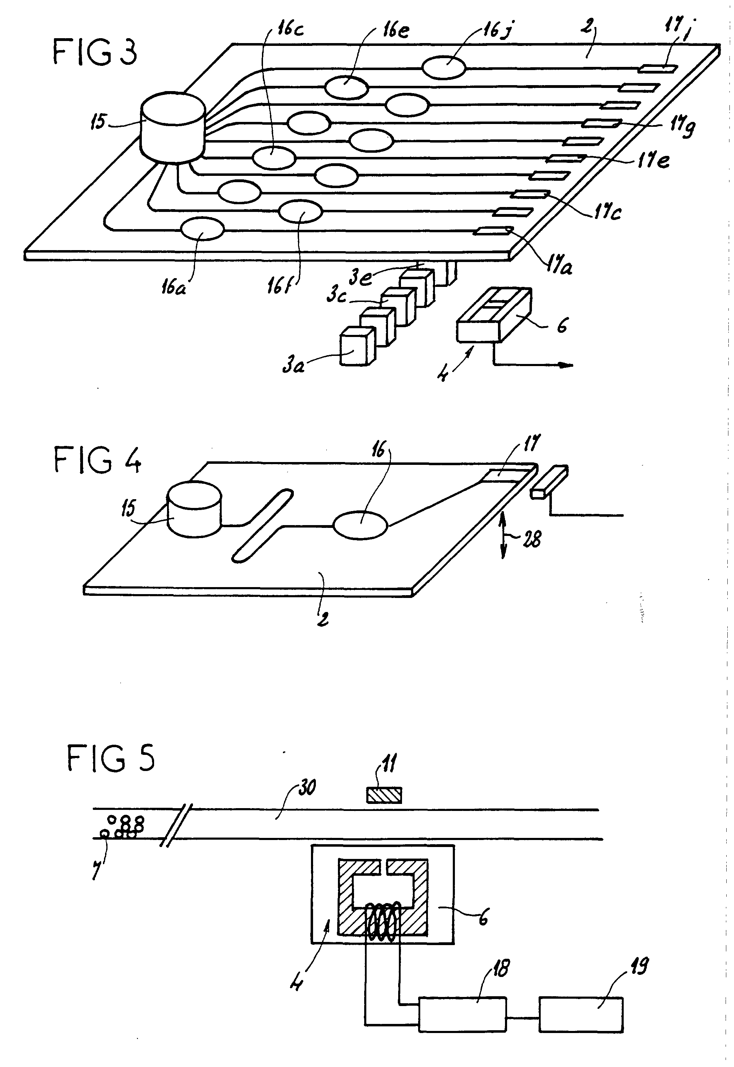

Le dispositif représenté à la figure 3 diffère de celui représenté à la figure 2, en ce qu'il consiste en une carte, permettant à partir d'un seul et même échantillon biologique d'effectuer plusieurs déterminations biologiques ou biochimiques. A cette fin, le support 2 consiste en une carte sculptée en volume, laquelle comprend :

- un puits de réception 15 de l'échantillon en phase liquide, une pluralité de puits d'incubation 16a à 16j, reliés en parallèle au seul puits de réception 15, formant chacun une première zone pour recevoir une quote-part de l'échantillon, le réactif, et le mélange intermédiaire une fois formé ;
- une pluralité de puits de lecture 17a à 17j reliés respectivement aux différents puits d'incubation 16a à 16j, formant chacun une deuxième zone de séparation d'un agrégat de mesure ;

- une pluralité d'aimants 3a à 3j disposés en relation avec la pluralité de canaux reliant respectivement les puits de réception aux puits de lecture, pour le transfert des complexes intermédiaires ;
- et un seul et même capteur 6 électromagnétique, susceptible de se déplacer, devant les différents puits de lecture 17a à 17j respectivement.

Le dispositif représenté à la figure 4 diffère de ceux représentés respectivement aux figures 2 et 3, par le fait que :

- sur la carte ou pochette formant le support 2, il existe un seul puits d'incubation 16 contenant, de manière préconditionnée, le réactif en phase liquide ;
- le puits de réception 15 est relié au puits d'incubation 16, par un canal étanche vis-à-vis de l'échantillon en phase liquide et du réactif en phase liquide, lorsque le support 2 n'est pas mis en vibration à plat selon la direction 28, et laissant passer ce même échantillon lorsque le support 2 est mis en vibration selon cette même direction ;
- et il existe un seul et même puits de lecture 17, relié au puits d'incubation 16 par un canal droit, alors que le canal reliant les puits 15 et 16 a une forme à plat en zig-zag.

Conformément à la figure 5, et selon un autre mode d'exécution de la présente invention, le moyen de confinement et séparation des complexes métalliques est un conduit de circulation 30 du mélange intermédiaire, par exemple un conduit capillaire, disposé devant le capteur électromagnétique 6, ou moyen de mesure de la masse ferro-magnétique de l'agrégat de mesure, et dont la section est adaptée à une détection de ladite masse par ledit capteur.

Selon ce mode d'exécution, on confine et sépare donc les complexes métalliques par circulation du mélange intermédiaire selon une veine liquide passant devant le capteur électromagnétique 6, et dont la section est adaptée à une détection de la masse ferro-magnétique de l'agrégat de mesure 7 par le capteur 6.

**Revendications**

1. Procédé pour la détermination qualitative et/ou quantitative d'un analyte dans un échantillon, selon lequel :

   **i)** on dispose dudit échantillon en phase liquide, dont l'analyte comprend un ligand ayant au moins un site de reconnaissance spécifique d'un anti-ligand ;
   **ii)** on dispose d'au moins un réactif comprenant des particules métalliques réactives en suspension en phase liquide, super-paramagnétiques, chaque particule réactive comprenant un noyau métallique sur lequel est fixé directement ou indirectement au moins undit anti-ligand ;
   **iii)** on met en contact, notamment par incubation, l'échantillon et le réactif, pour obtenir en phase liquide un mélange intermédiaire de complexes métalliques des particules réactives ayant réagi avec l'analyte, et des particules réactives résiduelles n'ayant pas réagi ;
   **iv)** on applique un champ magnétique au mélange intermédiaire ;
   **v)** on détecte les complexes métalliques, pour obtenir un signal de détection représentatif de la présence et/ou de la quantité desdits complexes, corrélé avec la présence et/ou la quantité d'analyte présente à l'origine dans l'échantillon,

   **caractérisé en ce que,** en combinaison :

   **a)** selon l'étape (iv), avec le champ magnétique appliqué au mélange intermédiaire, on confine les complexes métalliques des particules réactives ayant réagi avec l'analyte, selon un agrégat de mesure, et on sépare, éventuellement simultanément, ledit agrégat des particules résiduelles n'ayant pas réagi ;
   **b)** selon l'étape (v), on détecte l'agrégat de mesure séparé, en mesurant sa masse ferro-magnétique, représentative de la présence et/ou de la quantité totale desdits complexes métalliques.

2. Procédé selon la revendication 1, caractérisé en ce qu'on sépare l'agrégat de mesure du milieu intermédiaire dans lequel demeurent les particules résiduelles n'ayant pas réagi, en déposant, notamment déplaçant le champ magnétique servant au confinement des complexes métalliques, à partir et en dehors dudit milieu intermédiaire.

3. Procédé selon la revendication 2, caractérisé en ce qu'on confine et on sépare les complexes métalliques, par extraction de l'agrégat de mesure d'une première zone contenant le mélange intermédiaire, et transfert dudit agrégat vers une deuxième zone.

4. Procédé selon la revendication 3, caractérisé en ce que l'extraction et le transfert de l'agrégat de mesure, de la première zone vers la deuxième zone, s'effectuent sous l'effet d'un champ magnétique, focalisant les lignes magnétiques sur ledit agrégat, éventuellement mobile selon la direction de transfert.

5. Procédé selon la revendication 1, caractérisé en ce que le transfert de l'agrégat de mesure, de la première zone vers la deuxième zone, s'effectue à plat, sur un support imperméable vis-à-vis du mélange intermédiaire au moins.

6. Procédé selon la revendication 1, caractérisé en ce qu'on sépare l'agrégat de mesure du milieu intermédiaire dans lequel demeurent les particules résiduelles n'ayant pas réagi, en fixant le champ magnétique servant au confinement des complexes métalliques, et en déplaçant le milieu intermédiaire contenant les particules résiduelles à l'écart de l'agrégat de mesure.

7. Procédé selon la revendication 6, caractérisé en ce qu'on fait circuler le milieu intermédiaire par rapport au champ magnétique servant au confinement des complexes métalliques, demeurant fixe.

8. Procédé selon la revendication 7, caractérisé en ce qu'on fait circuler le mélange intermédiaire selon une veine liquide passant devant le moyen de mesure de la masse ferro-magnétique de l'agrégat de mesure, et dont la section est adaptée à une détection de ladite masse ferro-magnétique par ledit moyen de mesure.

9. Procédé selon la revendication 6, caractérisé en ce qu'on prélève le milieu intermédiaire, notamment par pompage ou absorption dans un milieu absorbant, par rapport à l'agrégat retenu par le champ magnétique de confinement des complexes métalliques, demeurant fixe.

10. Procédé selon la revendication 1, caractérisé en ce qu'on mesure la masse ferro-magnétique, en présentant l'agrégat de mesure devant un capteur électro-magnétique générant un signal électrique de mesure, représentatif de ladite masse ferro-magnétique.

11. Procédé selon la revendication 10, caractérisé en ce que l'agrégat de mesure est sollicité, devant le moyen de mesure, par des oscillations périodiques, notamment des vibrations.

12. Procédé selon les revendications 5 et 11, caractérisé en ce que, devant le capteur électromagnétique, le support plat est soumis à des oscillations périodiques, notamment vibrations, par exemple sous l'effet d'un jet gazeux rencontrant la tranche dudit support plat.

13. Procédé selon la revendication 10, caractérisé en ce qu'on applique un champ magnétique auxiliaire, au niveau du capteur électromagnétique, augmentant la masse magnétique de l'agrégat de mesure.

14. Dispositif (1) pour la détermination qualitative et/ou quantitative d'un analyte dans un échantillon, comprenant :

   i) un support (2) pour recevoir ledit échantillon en phase liquide, dont l'analyte comprend un ligand ayant au moins un site de reconnaissance spécifique d'un anti-ligand ;
   ii) une source d'au moins un réactif comprenant des particules métalliques réactives en suspension en phase liquide, super-paramagnétiques, chaque particule réactive comprenant un noyau métallique sur lequel est fixé directement ou indirectement au moins undit anti-ligand ;
   iii) des moyens de mise en contact, notamment par incubation, de l'échantillon et du réactif, pour obtenir en phase liquide un mélange intermédiaire de complexes métalliques des particules réactive ayant réagi avec l'analyte, et des particules réactives résiduelles n'ayant pas réagi ;
   iv) un moyen (3) d'application d'un champ magnétique au mélange intermédiaire ;
   v) un moyen (4) de détection des complexes métalliques séparés, pour obtenir un signal (5), de détection représentatif de la présence et/ou de la quantité desdits complexes, corrélé avec la présence et/ou la quantité d'analyte présente à l'origine dans l'échantillon,

   caractérisé en ce que, en combinaison :

   a) le moyen (3) d'application; du champ magnétique selon (iv) comprend ou sert à la fois de moyen de confinement des complexes métalliques des particules métalliques ayant réagi avec l'analyte, selon un agrégat de mesure, et de moyen de séparation dudit agrégat, demeurant sur ledit support (2), des particules résiduelles n'ayant pas réagi ;
   b) le moyen ((4) de détection selon (v) comprend, d'une part un moyen de mesure (6) de la masse ferro-magnétique de l'agrégat de mesure séparé, générant un signal de mesure représentatif de la présence et/ou de la quantité totale desdits complexes métalliques, et d'autre part un moyen de présentation dudit support (2) avec l'agrégat de mesure, devant le moyen de mesure.

15. Dispositif selon la revendication 14, caractérisé en ce que le moyen de mesure comprend un capteur (6) électromagnétique générant un signal électrique de mesure, représentatif de la masse ferro-magnétique de l'agrégat de mesure séparé.

16. Dispositif selon la revendication 14, caractérisé en ce qu'il comprend sur un même support (2), une première zone (8) pour recevoir ledit mélange intermédiaire, une deuxième zone (9) de séparation de l'agrégat de mesure, et des moyens de transfert (3) dudit agrégat de mesure de la première zone à la deuxième zone.

17. Dispositif selon la revendication 16, caractérisé en ce que le support (2) est plat, et imperméable vis-à-vis du mélange intermédiaire au moins.

18. Dispositif selon la revendication 14, caractérisé en ce que le support (2) comprend un puits de réception (15) de l'échantillon en phase liquide, au moins un puits d'incubation (16, 16a à 16j.) relié au puits (15) de réception, formant une première zone pour recevoir ledit mélange intermédiaire, au moins un puits de lecture (17, 17a à 17j.) relié au puits d'incubation, formant la deuxième zone de séparation de l'agrégat de mesure.

19. Dispositif selon la revendication 18, caractérisé en ce que le support (2) comprend une pluralité de puits d'incubation (16a à 16j) reliés d'un côté en parallèle à un seul puits de réception (15), et une pluralité de puits de lecture (17a à 17j) reliés respectivement aux différents puits d'incubation.

20. Dispositif selon la revendication 18, caractérisé en ce que le puits d'incubation (16) contient le réactif en phase liquide, et le puits de réception (15) est relié au puits d'incubation par un canal étanche vis-à-vis de l'échantillon en phase liquide et du réactif en phase liquide, lorsque le support (2) n'est pas mis en vibration, et laissant passer ledit échantillon lorsque le support (2) est mis en vibration.

21. Dispositif selon la revendication 14, caractérisé en ce qu'il comprend un conduit de circulation du mélange intermédiaire, disposé devant le moyen de mesure (6) de la masse ferro-magnétique de l'agrégat de mesure, et dont la section est adaptée à une détection de ladite masse ferro-magnétique par ledit moyen de mesure.

22. Dispositif selon la revendication 14, caractérisé en ce qu'un générateur (10) d'oscillations périodiques, notamment de vibrations, sollicitant l'agrégat de mesure, est associé au moyen de mesure (6), et consiste par exemple en un moyen de projection d'un flux gazeux tangentiel à l'encontre de la tranche du support (2).

23. Dispositif selon la revendication 14, caractérisé en ce qu'un générateur (11) magnétique auxiliaire est disposé au niveau du capteur électromagnétique (6), pour augmenter la masse magnétique de l'agrégat de mesure.

**Claims**

1. Method for the qualitative and/or quantitative determination of an analyte in a sample, according to which:

    i) said sample is available in liquid phase, in which the analyte comprises a ligand having at least one site for specific recognition of an anti-ligand;
    ii) at least one reagent is available comprising superparamagnetic metal particles which are reactive in suspension in liquid phase, each reactive particle comprising a metal core to which is attached, directly or indirectly, at least one said anti-ligand;
    iii) the sample and the reagent are brought into contact, in particular by incubation, in order to obtain, in liquid phase, an intermediate mixture of metal complexes of the reactive particles which have reacted with the analyte, and of the residual reactive particles which have not reacted;
    iv) a magnetic field is applied to the intermediate mixture;
    v) the metal complexes are detected in order to obtain a detection signal representative of the presence and/or of the amount of said complexes, which is correlated with the presence and/or the amount of analyte present at the start in the sample;

    **characterized in that,** in combination:

    **a)** according to step (iv), with the magnetic field applied to the intermediate mixture, the metal complexes of the reactive particles which have reacted with the analyte are confined, according to an measurable aggregate, and said aggregate of the residual particles which have not reacted is separated, optionally simultaneously;
    **b)** according to step (v), the separated measurable aggregate is detected, by measuring its ferromagnetic mass representative of the presence and/or of the total amount of said metal complexes.

2. Method according to Claim 1, characterized in that the measurable aggregate of the intermediate medium in which the residual particles which have not reacted remain is separated by applying, in particular displacing the magnetic field which is used to confine the metal complexes, from and outside of said intermediate medium.

3. Method according to Claim 2, characterized in that the metal complexes are confined and separated by extracting the measurable aggregate from a first region containing the intermediate mixture, and transferring said aggregate to a second region.

4. Method according to Claim 3, characterized in that the extraction and the transfer of the measurable aggregate, from the first region to the second region, are carried out under the effect of a magnetic field, focusing the magnetic lines on said aggregate, optionally mobile according to the direction of transfer.

5. Method according to Claim 1, characterized in that the transfer of the measurable aggregate, from the first region to the second region, is carried out flat, on a support which is impermeable with respect to the intermediate mixture at least.

6. Method according to Claim 1, characterized in that the measurable aggregate of the intermediate medium in which the residual particles which have not reacted remain is separated by fixing the magnetic field which is used to confine the metal complexes and displacing the intermediate medium containing the residual particles away from the measurable aggregate.

7. Method according to Claim 6, characterized in that the intermediate medium is circulated with respect to the magnetic field which is used to confine the metal complexes, and which remains fixed.

8. Method according to Claim 7, characterized in that the intermediate mixture is circulated according to a liquid stream which passes in front of the means of measuring the ferromagnetic mass of the measurable aggregate, and the section of which is suitable for detection of said ferromagnetic mass by said measuring means.

9. Method according to Claim 6, characterized in that the intermediate medium is removed, in particular by pumping or absorption in an absorbent medium, with respect to the aggregate retained by the magnetic field for confining the metal complexes, which remains fixed.

10. Method according to Claim 1, characterized in that the ferromagnetic mass is measured by presenting the measurable aggregate in front of an electromagnetic sensor which generates a measurable electric signal, representative of said ferromagnetic mass.

11. Method according to Claim 10, characterized in that the measurable aggregate is solicited, in front of the measuring means, by periodic oscillations, in particular vibrations.

12. Method according to Claims 5 and 11, characterized in that, in front of the electromagnetic sensor, the flat support is subjected to periodic oscillations, in particular vibrations, for example under the effect of a gaseous jet coming up against the edge of said flat support.

13. Method according to Claim 10, characterized in that an auxiliary magnetic field is applied, in the region of the electromagnetic sensor, which increases the magnetic mass of the measurable aggregate.

14. Device (1) for the qualitative and/or quantitative determination of an analyte in a sample, comprising:

   i) a support (2) for receiving said sample in liquid phase, in which the analyte comprises a ligand having at least one site for specific recognition of an anti-ligand;
   ii) a source of at least one reagent comprising superparamagnetic metal particles which are reactive in suspension in liquid phase, each reactive particle comprising a metal core to which is attached, directly or indirectly, at least one said anti-ligand;
   iii) means for bringing the sample and the reagent into contact, in particular by incubation, in order to obtain, in liquid phase, an intermediate mixture of metal complexes of the reactive particles which have reacted with the analyte, and of the residual reactive particles which have not reacted;
   iv) a means (3) of applying a magnetic field to the intermediate mixture;
   v) a means (4) of detecting the separated metal complexes, in order to obtain a detection signal (5) representative of the presence and/or of the amount of said complexes, correlated with the presence and/or the amount of analyte present at the start in the sample,

characterized in that, in combination:

   a) the means (3) of applying the magnetic field according to (iv) comprises, or is used as, both a means of confining the metal complexes of the metal particles which have reacted with the analyte, according to an measurable aggregate, and a means of separating said aggregate, which remains on said support (2), from the residual particles which have not reacted;
   b) the detection means (4) according to (v) comprises, on the one hand, a means of measuring (6) the ferromagnetic mass of the separated measurable aggregate, which generates a measurable signal representative of the presence and/or of the total amount of said metal complexes and, on the other hand, a means of presenting, in front of the measuring means, said support (2) with the measurable aggregate.

15. Device according to Claim 14, characterized in that the measuring means comprises an electromagnetic sensor (6) which generates a measurable electric signal representative of the ferromagnetic mass of the separated measurable aggregate.

16. Device according to Claim 14, characterized in that it comprises, on the same support (2), a first region (8) for receiving said intermediate mixture, a second region (9) for separating the measurable aggregate, and means of transferring (3) said measurable aggregate from the first region to the second region.

17. Device according to Claim 16, characterized in that the support (2) is flat and impermeable with respect to the intermediate mixture at least.

18. Device according to Claim 14, characterized in that the support (2) comprises a well for receiving (15) the sample in liquid phase, at least one incubation well (16, 16a to 16j) connected to the reception well (15), forming a first region for receiving said intermediate mixture, and at least one reading well (17, 17a to 17j) connected to the incubation well, forming the second region for separating the measurable aggregate.

19. Device according to Claim 18, characterized in that the support (2) comprises a plurality of incubation wells (16a to 16j) connected on one side, in parallel, to a single reception well (15), and a plurality of reading wells (17a to 17j) connected, respectively, to the various incubation wells.

20. Device according to Claim 18, characterized in that the incubation well (16) contains the reagent in liquid phase, and the reception well (15) is connected to the incubation well via a channel which is tight with respect to the sample in liquid phase and to the reagent in liquid phase, when the support (2) is not vibrated, and which allows said sample to pass when the support (2) is vibrated.

21. Device according to Claim 14, characterized in that it comprises a tube for circulating the intermediate mixture, which is placed in front of the means of measuring (6) the ferromagnetic mass of the measurable aggregate, and the section of which is suitable for detection of said ferromagnetic mass by said measuring means.

22. Device according to Claim 14, characterized in that a generator (10) of periodic oscillations, in particular of vibrations, which solicits the measurable aggregate, is combined with the measuring means (6) and consists, for example, of a means for projecting a tangential gaseous flow against the edge of the support (2).

23. Device according to Claim 14, characterized in that an auxiliary magnetic generator (11) is placed in the region of the electromagnetic sensor (6), in order to increase the magnetic mass of the measurable aggregate.

**Patentansprüche**

1. Verfahren, um in einer Probe einen Analyt qualitativ und/oder quantitativ zu bestimmen, nach dem:

i) die Probe in flüssiger Phase bereitgestellt wird, wobei deren Analyt einen Liganden mit zumindest einer spezifischen Erkennungsstelle für einen Anti-Liganden aufweist;
ii) zumindest ein Reagenz bereitgestellt wird, das super-paramagnetische, in flüssiger Phase in Suspension befindliche reaktive Metallpartikel aufweist, wobei jeder reaktive Partikel einen Metallkern aufweist, auf dem direkt oder indirekt zumindest ein Anti-Ligand fixiert ist;
iii) die Probe und das Reagenz, insbesondere durch Inkubation, in Kontakt gebracht wird, um in flüssiger Phase ein intermediäres Gemenge aus Metallkomplexen aus reaktiven Partikeln, die mit dem Analyt reagiert haben, und aus verbleibenden reaktiven Partikeln, die nicht reagiert haben, zu erhalten;
iv) an das intermediäre Gemenge ein Magnetfeld angelegt wird, und
v) die Metallkomplexe nachgewiesen werden, um ein für das Vorhandensein und/oder die Menge der Komplexe repräsentives Nachweissignal zu erhalten, das mit dem Vorhandensein und/oder der Menge des Analyts, der in der ursprünglichen Probe vorhanden ist, korreliert,

**dadurch gekennzeichnet, daß** in Kombination:

a) entsprechend Schritt (iv) mit dem an dem intermediären Gemenge angelegten Magnetfeld die Metallkomplexe aus reaktiven Partikeln, die mit dem Analyt reagiert haben, zu einem Meßkonglomerat zusammengeschlossen werden, und, gegebenenfalls gleichzeitig, das Konglomerat von den verbleibenden Partikeln, die nicht reagiert haben, abgetrennt wird, und
b) entsprechend Schritt (v) das abgetrennte Meßkonglomerat nachgewiesen wird, indem dessen für das Vorhandensein und/oder die Gesamtmenge der Metallkomplexe repräsentative ferromagnetische Masse gemessen wird.

2. Verfahren nach Anspruch 1, dadurch gekennzeichnet, daß das Meßkonglomerat dadurch vom intermediären Medium, in dem die nicht reagierten verbleibenden Partikel enthalten sind, getrennt wird, indem das Magnetfeld, das zum Zusammenschließen der Metallkomplexe dient, von und aus dem intermediären Medium abgesetzt, insbesondere verschoben wird.

3. Verfahren nach Anspruch 2, dadurch gekennzeichnet, daß die Metallkomplexe durch Abziehen des Meßkonglomerats von einer ersten Zone, die das intermediäre Gemenge enthält, und Überführen des Konglomerats zu einer zweiten Zone zusammengeschlossen und abgetrennt werden.

4. Verfahren nach Anspruch 3, dadurch gekennzeichnet, daß das Abziehen und das Überführen des Meßkonglomerats von der ersten Zone zu der zweiten Zone unter Wirkung eines gegebenenfalls in Überführungsrichtung beweglichen Magnetfeldes erfolgt, dessen magnetische Feldlinien auf das Konglomerat konzentriert werden.

5. Verfahren nach Anspruch 1, dadurch gekennzeichnet, daß die Überführung des Meßkonglomerats von der ersten Zone zu der zweiten Zone eben auf einem Träger durchgeführt wird, der zumindest gegenüber dem intermediären Gemenge undurchlässig ist.

6. Verfahren nach Anspruch 1, dadurch gekennzeichnet, daß das Meßkonglomerat von dem intermediären Medium, in dem die verbleibenden Partikel, die nicht reagiert haben, enthalten sind, dadurch getrennt werden, indem das Magnetfeld, das zum Zusammenschließen der Metallkomplexe dient, fixiert wird und daß das intermediäre Medium, das die verbleibenden Partikel enthält, von dem Meßkonglomerat weg verschoben wird.

7. Verfahren nach Anspruch 6, dadurch gekennzeichnet, daß man das intermediäre Medium in bezug auf das fixierte Magnetfeld, das zum Zusammenschließen der Metallkomplexe dient, zirkulieren läßt.

8. Verfahren nach Anspruch 7, dadurch gekennzeichnet, daß man das intermediäre Gemenge entlang eines flüssigen Stromes zirkulieren läßt, der vor dem Mittel zum Messen der ferromagnetischen Masse des Meßkonglomerats vorbeitritt, wobei dessen Querschnitt für einen Nachweis der ferromagnetischen Masse durch das Meßmittel angepaßt ist.

9. Verfahren nach Anspruch 6, dadurch gekennzeichnet, daß das intermediäre Medium im Vergleich zu dem Konglomerat, welches durch das die Metallkomplexe zusammenschließende, fixiert bleibende Magnetfeld zurückbehalten wird, insbesondere durch Abpumpen oder durch Aufsaugen in ein aufsaugendes Milieu entzogen wird.

10. Verfahren nach Anspruch 1, dadurch gekennzeichnet, daß die ferromagnetische Masse gemessen wird, indem das Meßkonglomerat vor einem elektromagnetischen Sensor präsentiert wird, der ein elektrisches Meßsignal generiert, das für die ferromagnetische Masse repräsentativ ist.

11. Verfahren nach Anspruch 10, dadurch gekennzeichnet, daß das Meßkonglomerat vor dem Meßmittel durch periodische Oszillationen, insbesondere Vibrationen, angeregt wird.

12. Verfahren nach den Ansprüchen 5 und 11, dadurch gekennzeichnet, daß der flache Träger vor dem elektromagnetischen Sensor periodischen Oszillationen, insbesondere Vibrationen, ausgesetzt wird, z.B. unter Wirkung eines Gasstrahls, der auf den Rand des flachen Trägers auftrifft.

13. Verfahren nach Anspruch 10, dadurch gekennzeichnet, daß im Bereich des elektromagnetischen Sensors ein magnetisches Hilfsfeld angelegt wird, das die magnetische Masse des Meßkonglomerats erhöht.

14. Vorrichtung (1) zur qualitativen und/oder quantitativen Bestimmung eines Analyts in einer Probe, die folgendes aufweist:

   i) einen Träger (2) zum Aufnehmen der Probe in flüssiger Phase, deren Analyt einen Liganden aufweist, welcher zumindest eine spezifische Erkennungsstelle eines Anti-Liganden hat;
   ii) eine Quelle von zumindest einem Reagenz, das in flüssiger Phase in Suspension befindliche, super-paramagnetische reaktive Metallpartikel aufweist, wobei jeder reaktive Partikel einen Metallkern aufweist, auf dem direkt oder indirekt zumindest ein Anti-Ligand fixiert ist;
   iii) Mittel zum Inkontaktbringen, insbesondere durch Inkubation, der Probe und des Reagenz, um in flüssiger Phase ein intermediäres Gemenge aus Metallkomplexen aus reaktiven Partikeln, die mit dem Analyt reagiert haben, und aus verbleibenden reaktiven Partikeln, die nicht reagiert haben, zu erhalten;
   iv) ein Mittel (3) zum Anlegen eines Magnetfeldes an das intermediäre Gemenge, und
   v) ein Mittel (4) zum Nachweisen von abgetrennten Metallkomplexen, um ein Nachweissignal (5) zu erhalten, das das Vorhandensein und/oder die Menge der Komplexe repräsentiert, und das mit dem Vorhandensein und/oder der Menge des Analyts in der ursprünglichen Probe korreliert,

dadurch gekennzeichnet, daß in Kombination:

a) die Mittel (3) zum Anlegen eines Magnetfeldes entsprechend (iv), Mittel zum Zusammenschließen der Metallkomplexe aus solchen Metallpartikeln, die mit dem Analyt zu einem Meßkonglomerat reagiert haben, und Mittel zum Abtrennen des auf dem Träger (2) zurückbleibenden Konglomerats, von verbleibenden Partikeln, die nicht reagiert haben, aufweisen oder unmittelbar dazu dienen, und
b) das Nachweismittel (4) entsprechend (v), einerseits ein Mittel zum Messen (6) der ferromagnetischen Masse des abgetrennten Meßkonglomerats aufweist, wobei dieses ein Meßsignal generiert, das das Vorhandensein und/oder die Gesamtmenge der Metallkomplexe repräsentiert, und andererseits ein Mittel zum Präsentieren dieses Trägers (2) mit dem Meßkonglomerat vor dem Meßmittel aufweist.

15. Vorrichtung nach Anspruch 14, dadurch gekennzeichnet, daß das Meßmittel einen elektromagnetischen Sensor (6) aufweist, der ein elektrisches Meßsignal generiert, das die ferromagnetische Masse des abgetrennten Meßkonglomerats repräsentiert.

16. Vorrichtung nach Anspruch 14, dadurch gekennzeichnet, daß sie auf dem selben Träger (2) eine erste Zone (8) zum Aufnehmen des intermediären Gemenges, eine zweite Meßkonglomerat-Abtrennzone (9), und Mittel zum Überführen (3) des Meßkonglomerats aus der ersten Zone zu der zweiten Zone aufweist.

17. Vorrichtung nach Anspruch 16, dadurch gekennzeichnet, daß der Träger (2) flach ist und zumindest gegenüber dem intermediären Gemenge undurchlässig ist.

18. Vorrichtung nach Anspruch 14, dadurch gekennzeichnet, daß der Träger (2) eine Aufnahmekammer (15) für die Probe in flüssiger Phase, zumindest eine Inkubationskammer (16, 16a bis 16j), die mit der Aufnahmekammer (15) verbunden ist, wodurch eine erste Zone zur Aufnahme des intermediären Gemenges ausgebildet wird, und zumindest eine Lesekammer (17, 17a bis 17j.), die mit der Inkubationskammer verbunden ist, wodurch die zweite Meßkonglomerat-Abtrennzone gebildet wird, aufweist.

19. Vorrichtung nach Anspruch 18, dadurch gekennzeichnet, daß der Träger (2) eine Vielzahl von Inkubationskammern (16a bis 16j), die auf einer Seite parallel verlaufend mit einer einzigen Aufnahmekammer (15) verbunden sind, und eine Vielzahl von Lesekammern (17a bis 17j), die mit entsprechend mehreren Inkubationskammern verbunden sind, aufweist.

20. Vorrichtung nach Anspruch 18, dadurch gekennzeichnet, daß die Inkubationskammern (16), die das Reagenz in flüssiger Phase enthalten, und die Aufnahmekammern (15) mit der Inkubationskammer über einen Kanal verbunden sind, der gegenüber der in flüssiger Phase befindlichen Probe und dem in flüssiger Phase befindlichen Reagenz dann verschlossen ist, wenn der Träger (2) nicht in Vibration versetzt ist, und der die Probe hindurchtreten läßt, wenn der Träger (2) in Vibration versetzt ist.

21. Vorrichtung nach Anspruch 14, dadurch gekennzeichnet, daß sie eine Leitung zur Zirkulation des intermediären Gemenges aufweist, die vor dem Mittel zum Messen (6) der ferromagnetischen Masse des Meßkonglomerats angeordnet ist, und deren Querschnitt für einen Nachweis der ferromagnetischen Masse durch das Meßmittel angepaßt ist.

22. Vorrichtung nach Anspruch 14, dadurch gekennzeichnet, daß ein Generator (10) von periodischen Oszillationen, insbesondere Vibrationen, die das Meßkonglomerat anregen, mit dem Meßmittel (6) verbunden ist, und z.B. aus einem Mittel zum Ausstoßen eines gasförmigen Stroms, der tangential gegen den Rand des Trägers (2) gerichtet ist, besteht.

23. Vorrichtung nach Anspruch 14, dadurch gekennzeichnet, daß ein magnetischer Hilfsgenerator (11) im Bereich des elektromagnetischen Sensors (6) angeordnet ist, um die magnetische Masse des Meßkonglomerats zu erhöhen.

FIG 1

FIG 2

FIG 3

FIG 4

FIG 5